# EUROPÄISCHE PATENTSCHRIFT

(11) **EP 4 115 812 B1**
(45) Veröffentlichungstag und Bekanntmachung des Hinweises auf die Patenterteilung: **28.01.2026**
(21) Anmeldenummer: 21183848.7
(22) Anmeldetag: 06.07.2021
(51) Int. Cl.: A61B 6/00, A61B 6/51

(54) **INTRAORAL-RÖNTGENSYSTEM MIT FUNKTIONSWEICHE IM RÖNTGENGERÄT ZUR VERWENDUNG UNTERSCHIEDLICHER SENSORTYPEN**
INTRAORAL X-RAY SYSTEM WITH FUNCTION SWITCH IN THE X-RAY UNIT FOR THE USE OF DIFFERENT SENSOR TYPES
SYSTÈME DE RADIOGRAPHIE INTRA-ORALE AVEC AIGUILLAGE FONCTIONNEL DANS L'APPAREIL DE RADIOGRAPHIE POUR L'UTILISATION DE DIFFÉRENTS TYPES DE CAPTEURS

(43) Veröffentlichungstag der Anmeldung: 11.01.2023
(73) Patentinhaber: DENTSPLY SIRONA Inc., York, PA 17401 (US); Sirona Dental Systems GmbH, 64625 Bensheim (DE)
(72) Erfinder: Schulze-Ganzlin, Ulrich, 64653 Lorsch (DE); Lindenberg, Kai, 64625 Bensheim (DE)
(74) Vertreter: Aldridge, Henry Alexander

(56) Entgegenhaltungen:
- EP-A1- 3 673 810
- WO-A1-96/03917
- US-A1- 2014 010 350
- US-A1- 2016 262 715
- US-A1- 2017 188 987

## Beschreibung

### TECHNISCHES GEBIET DER ERFINDUNG

Die vorliegende Erfindung bezieht sich auf ein Intraoral-Röntgensystem mit automatischer Belichtungskontrolle (AEC) Funktionalität, bestehend aus einem intraoralen Röntgensensor und einem dentalen Röntgengerät.

### HINTERGRUND DER ERFINDUNG

Die automatischen Belichtungskontrolle, nämlich die AEC (automatic exposure control)-Technik ist vorbekannt, auch im Dentalbereich für intraorales Röntgen mit digitalen Röntgenbildempfängern, siehe z.B. EP1859659A1 und US 6,898,268 B2.

Röntgenbilddetektoren haben für typische Belichtungssituationen in der Intraoral-Röntgendiagnostik einen optimierten Dynamikumfang. Unterbelichtete Aufnahmen zeichnen sich durch erhöhtes Rauschen aus, überbelichtete Bereiche können aufgrund von Sättigungseffekten der Pixel das Signal nicht mehr auflösen. Mit Hilfe der AEC-Technik kann die Sättigungsgrenze durch Mehrfachbelichtungen und Aufsummierung der Einzelaufnahmen aufgelöst werden. Durch das Senken der Sättigungsgrenze kann der Röntgenbildempfänger für niedrig-Belichtungen optimiert werden. Mit dieser Konstellation kann der Anwender indikationsabhängig die jeweils erforderliche Mindestbildqualität gezielt festlegen um die Patientendosis so niedrig wie möglich zu halten.

Mit einem Scout Shot kann die Belichtungssituation ausgemessen werden. Dazu werden Informationen zum Belichtungsgrad dieser ersten Aufnahme herangezogen. Diese geben an, welcher Maximalwert einer bestimmten Anzahl an Pixeln erreicht oder überschritten wurde. Es kann auch ein relativer Wert mit Bezug zu einem maximal zulässigen Wert sein, z.B. in Prozent zur Sättigungsgrenze. Informationen zum Belichtungsgrad kann auch das jeweilige Histogramm beinhalten.

Bei AEC-Röntgen Systemen mit Scout Shot ist eine schnelle Auswertung der vorbelichteten Aufnahmen zur Bestimmung der vollständigen Röntgenexposition erforderlich, um Bewegungsartefakte zu reduzieren, die durch Bewegungen der Aufnahmegeometrie während der Aufnahmesession hervorgerufen werden können. Bisher erfolgt die Auswertung des Belichtungsgrades des Scout Shots losgelöst vom Intraoral-Röntgensensor (im Folgenden auch kurz "Sensor" genannt) durch eine Auswerteeinheit, die im Röntgengerät (oder im angeschlossenen Rechner) angeordnet ist. Der bisherige Ablauf ist wie folgt:
1. Sensor erfasst den 1.ten Shot
2. Sensor überträgt teilweise oder vollständig den 1.ten Shot ans Röntgengerät
3. Auswerteeinheit analysiert 1. Shot im Röntgengerät
4. Auswerteeinheit legt Belichtungsgrad für den 2.ten Shot fest
5. Sensor erfasst den 2.ten Shot
6. Sensor überträgt den 2.ten Shot ans Röntgengerät
7. Auswerteeinheit berechnet im Röntgengerät aufsummiertes Bild aus den 1.ten Shot und 2.ten Shot

Im Allgemeinen ist das Intraoral-Röntgengerät mit AEC-Funktionalität mit einem Rechner oder einer Cloud verbunden. Der in Aufnahmebereitschaft versetzte Röntgensensor erfasst das Röntgenbild. Nach der Belichtung werden die Röntgenbilddaten aus dem Röntgendetektor ausgelesen und an den Rechner weitergeleitet.

Es wird Bezug genommen auf US2016/262715 A1, WO96/03917 A1, US2017/188987 A1, EP3673810 A1, US2014010350 A1 welche Intraoral-Röntgensensoren betreffen.

### OFFENBARUNG DER ERFINDUNG

Ein Ziel der vorliegenden Erfindung ist, ein Intraoral-Röntgensystem mit Modus-weiche bereitzustellen, dass die Verwendung unterschiedlicher Sensortypen ermöglicht, darunter ein Intraoral-Röntgensensor mit oder ohne AEC-Funktionalitäten.

Diese Ziele werden durch ein Intraoral-Röntgensystem nach Anspruch 1 erreicht. Die Gegenstände der abhängigen Ansprüche beziehen sich auf Weiterentwicklungen und bevorzugte Ausführungsformen.

Das erfindungsgemäße Intraoral-Röntgensystem ist in den beigefügten Ansprüchen beschrieben.

In der nachfolgenden Beschreibung werden unterschiedliche Röntgensensortypen und die dazu gehörigen Modi erklärt.

### AEC Plus Modus und ein Intraoral-Röntgensensor mit Belichtungsgrad Erfassung zur Verwendung mit einem Röntgen System mit AEC-Funktionalität:

Gemäß der vorliegenden Erfindung wird in dem AEC Plus Modus (Modus (M")) die Analyse des Scout Bildes oder Video Streams hinsichtlich des Belichtungsgrades vom IO-Röntgensensor durchgeführt, *nicht* vom IO-Röntgengerät oder anderen Systemkomponenten, wie z.B. einem Rechner. Dazu kann der Video Stream z.B. als unabhängige Folge von Einzelbildern verwendet werden.

Wird die oben genannte Auswertung in den IO-Röntgensensor (im Folgenden auch kurz "Sensor" genannt) verlagert reduziert sich der Datentransfer und die Komplexität auf der Seite des IO-Röntgengerätes. Der erfindungsgemäße Ablauf ist dann z.B. wie folgt:
1. Sensor erfasst den 1.ten Shot (Scout Shot);
2. Auswerteeinheit (z.B. eine Exposition Analyse-Einheit des Sensors) analysiert 1. Shot und stellt Informationen zum Belichtungsgrad zusammen;
3. Informationen zum Belichtungsgrad, sowie ggf. Sensoreigenschaften, werden an Entscheidungseinheit außerhalb des Sensors übertragen;
4. Entscheidungseinheit bestimmt anhand dieser und weiterer Informationen die Belichtungsparameter weiterer Shots;
5. Entsprechend dieser Entscheidung erfolgen keine, eine oder weitere Belichtungen, die der Sensor erfasst;
6. Sensor überträgt Einzelaufnahmen und/oder (optional) mindestens eine summierte Aufnahme an das IO-Röntgengerät bzw. PC.

Ein wesentliches Merkmal der vorliegenden Erfindung ist, dass die dem IO-Röntgensensor zugeordnete Elektronik (z.B. Exposition Analyse-Einheit) in der Lage ist Informationen zum Belichtungsgrad des Scout Bildes oder Video Streams zu erfassen und diese Information dem IO-Röntgengerät mitzuteilen. Dadurch reduziert sich der Initialisierungsaufwand und der Ablauf wird beschleunigt.

Das IO-Röntgengerät, insbesondere die Entscheidungseinheit des IO-Röntgengeräts oder anderer Systemkomponenten, verwendet diese Information des Belichtungsgrades und andere Vorgaben vorzugsweise sensorspezifische Eigenschaften, geforderte Bildqualität, Indikation, kV-Anpassung bei Dual Energy, erhöhte Dynamikbereich (HDR), Dosisabhängiges Rauschverhalten, usw. um daraus Belichtungsparameter z.B. Anzahl der Aufnahmen, Belichtungszeit, Röhrenstrom und Röhrenspannung, für nachfolgende Belichtung festzulegen. Dies könnte eine weitere Belichtung, aber auch mehrere oder keine weitere Belichtung sein.

Im Fall eines Video Streams wird bei Erreichen eines von der Entscheidungseinheit des Röntgengeräts berechneten Wertes die Belichtung vom Röntgengerät vorzugsweise abgebrochen.

Als mögliche Unterbringungsorte für die Auswerteeinheit (z.B. Exposition Analyse-Einheit) bietet sich vorzugsweise der IO-Röntgensensorkopf oder der IO-Röntgensensorstecker an. Alternativ könnte der genannte Sensorkopf mit dem IO-Röntgengerät drahtlos verbunden werden, so dass kein Stecker benötigt wird.

### AEC Modus und ein Intraoral-Röntgensensor ohne Belichtungsgrad Erfassung zur Verwendung mit einem Röntgensystem mit AEC-Funktionalität.

Gemäß der vorliegenden Erfindung wird, in dem AEC Modus (Modus (M')), die Analyse des Scout Bildes oder Video Streams hinsichtlich des Belichtungsgrades *nur* vom Röntgengerät oder von weiteren Systemkomponenten durchgeführt. Der AEC Plus Modus unterscheidet sich vom AEC Modus, in dem die oben erwähnte Auswertung im Röntgensensor stattfindet und nicht im IO-Röntgengerät.

### Standard Modus und Intraoral-Röntgensensor ohne Belichtungsgrad Erfassung zur Verwendung mit Röntgensystem ohne AEC-Funktionalität

Gemäß der vorliegenden Erfindung wird in dem Standard Modus keine Analyse des Scout Bildes oder Video Streams hinsichtlich des Belichtungsgrades weder vom Intraoral-Röntgensensor noch vom Röntgengerät/System durchgeführt.

### KURZBESCHREIBUNG DER ZEICHNUNGEN

In der nachfolgenden Beschreibung wird die vorliegende Erfindung anhand von Ausführungsbeispielen unter Bezugnahme auf die Zeichnungen näher erläutert, wobei
Abb.1 - ist eine schematische Darstellung eines Intraoral-Röntgensystems, an dem ein Standard IO-Röntgensensor zum Betrieb im Standard Modus angeschlossen ist;
Abb.2 - ist eine schematische Darstellung eines Intraoral-Röntgensystems mit AEC Funktionalität an dem ein IO-Röntgensensor zum Betrieb im AEC Modus angeschlossen ist;
Abb.3 - ist eine schematische Darstellung eines Intraoral-Röntgensystems mit AEC Funktionalität an dem ein IO-Röntgensensor zum Betrieb im AEC Plus Modus angeschlossen ist.

Die in den Abbildungen stark umrandeten Funktionsblöcke sind im jeweiligen Modus nicht zwingend aktiviert.

Die in den Zeichnungen gezeigten Referenznummern bezeichnen die unten aufgeführten Elemente, auf die in der nachfolgenden Beschreibung der beispielhaften Ausführungsformen Bezug genommen wird.
1. Intraoral-Röntgensensor (Sensortyp für Standard Modus)
   1' Intraoral-Röntgensensor (Sensortyp für AEC Modus)
   1" Intraoral-Röntgensensor (Sensortyp für AEC Plus Modus)
   1.1 Kommunikationsschnittstelle
   1.2 Exposition Analyse-Einheit
   1.3 Bildgebender Röntgendetektor
   1.4 Steuerung
   1.5 Sensorspezifische Eigenschaften
   1.6 Bildspeicher
2. Röntgengerät
   2.1.1 Kommunikationsschnittstelle
   2.1.2 Kommunikationsschnittstelle
   2.1.3 Kommunikationsschnittstelle
   2.2 Entscheidungs-Einheit
   2.3 Röntgen Strahler
   2.4 Steuerung
   2.5 Kommunikationsschnittstelle
   2.6 Stromversorgung und Hochspannungsquelle
   2.7 Benutzerschnittstelle
   2.8 Exposition Analyse-Einheit
   2.9 Bildspeicher
   2.10 Funktionsweiche
3. Intraoral-Röntgensystem
   4.1 Rechner
   4.2 Benutzerschnittstelle und Bildschirm
5. Netzwerk
6. Cloud

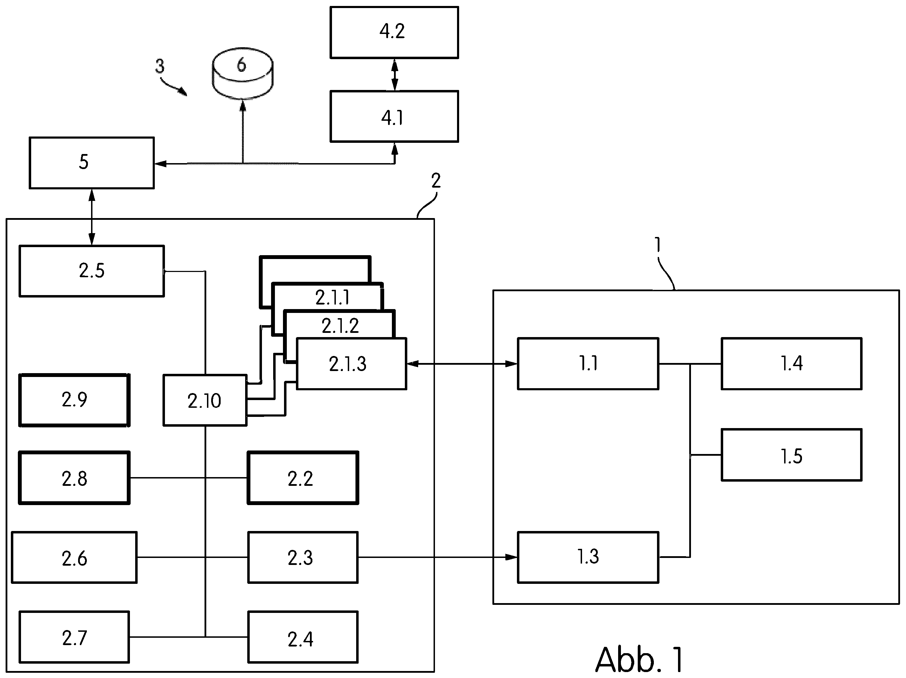

Wie in den Abbildungen 1 bis 3 unterschiedlicher Ausführungsformen gezeigt wurde, umfasst das Intraoral-Röntgensystem (3) ein Intraoral-Röntgengerät (2) das mit einer Automatischen Belichtungskontrolle (AEC) Funktionalität ausgestattet ist, und mit einer Cloud (6) und einem Rechner (4.1) verbindbar ist, wobei an dem Röntgengerät (2) mindestens ein Intraoral-Röntgensensor (1; 1'; 1") anschließbar ist, und das Röntgengerät (2) eine Funktionsweiche (2.10) aufweist welche in einem initialen Identifikationsprozess den **Sensortyp** des mindestens einem angeschlossenen Intraoral-Röntgensensors (1;1';1") erkennt, und einen dazugehörigen **Modus** (M; M'; M") aktiviert. Das Intraoral-Röntgensystem (3) umfasst die folgenden Modi (M; M'; M"): ein Modus (M") zur Intraoral-Röntgensensorseitigen Erfassung eines Belichtungsgrades, ein Modus (M') zur Intraoral-Röntgengerät-, Rechner- oder Cloudseitigen Erfassung eines Belichtungsgrades, und optional ein Modus (M) ohne Erfassung eines Belichtungsgrades.

### AEC Plus Modus

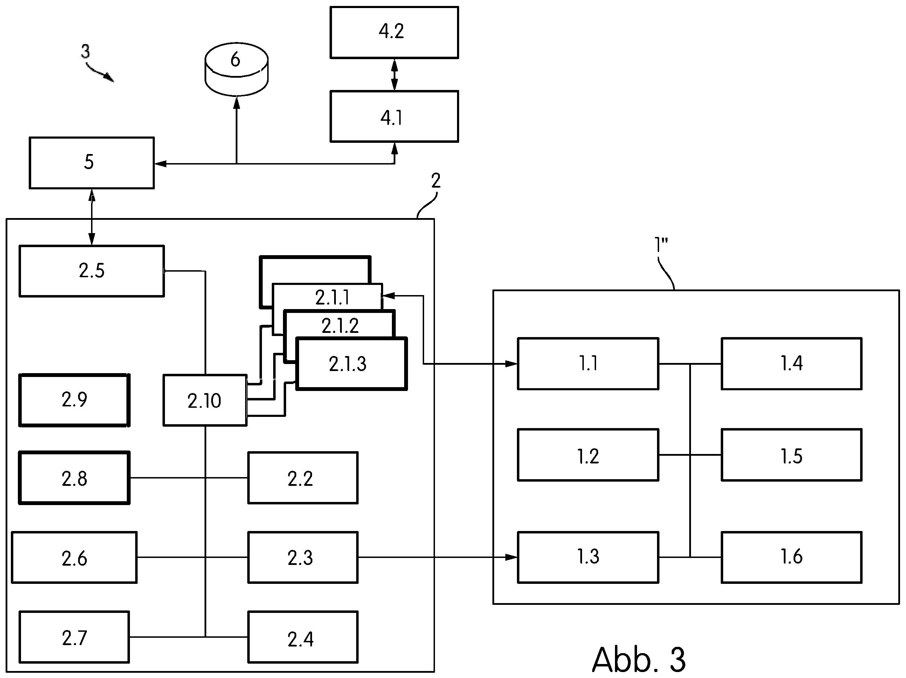

Im Folgenden wird der Modus (M") erklärt, welcher den eingangsseitig erklärten AEC Plus Modus entspricht: Abb.3 zeigt eine schematische Darstellung eines Intraoral-Röntgensystems (3) gemäß der Erfindung. Das Intraoral-Röntgensystem (3) hat ein Intraoral-Röntgengerät (2), das mit einem Intraoral-Röntgensensor (1") über die Kommunikationsstellen (2.1.1; 1.1) verbunden ist. Die Verbindung ist vorzugsweise eine Kabelverbindung. Der IO-Röntgensensor (1") bestehet aus einem Sensorkopf (Gehäuse), Kabel und Stecker. Über diesen Stecker ist der IO-Röntgensensor (1") mit dem Intraoral-Röntgengerät (2) verbunden. Am IO-Röntgengerät (2) können vorzugsweise gleichzeitig weitere Röntgensensoren (1, 1',1") mit oder ohne AEC Funktionalität, unterschiedlichen Typs über deren Stecker angeschlossen sein. Alternativ kann eine drahtlose Verbindung benutzt werden. Der Intraoral-Röntgensensor (1") hat eine Kommunikationsschnittstelle (1.1), eine Exposition Analyse-Einheit (1.2), einen bildgebenden Röntgendetektor (1.3), eine Steuerung (1.4), einen Speicher (1.5), der sensorspezifische Eigenschaften beinhaltet, und einen Bildspeicher (1.6). Der Bildspeicher (1.6) speichert die vom bildgebenden Röntgendetektor (1.3) empfangene Bilddaten teilweise oder vollständig. Der Bildspeicher (1.6) ist nützlich falls sich Pixelbilddaten aus dem bildgebenden Röntgendetektor (1.3) nicht ohne Signalverlust zur Auswertung auslesen lassen oder ein Signalverlust aus Gründen der Strahlenhygiene nicht akzeptabel ist. Vorzugsweise kann der Röntgensensor (1") mit einer Batterie und einer drahtlosen Kommunikation ausgestattet sein. Dies erlaubt auf ein Kabel zu verzichten. Eine kabelgebundene Datenkommunikation und/oder Energieversorgung kann auch alternativ berücksichtigt werden. Hier bietet sich als Standard eine USB-Schnittstelle oder Power over Ethernet (PoE) an.

Der Intraoral-Röntgensensor (1") beinhaltet Komponenten für eine automatische Belichtungskontrolle (AEC) Funktionalität, die im Folgenden naher erklärt wird. Der vom bildgebender Röntgendetektor (1.3) empfangene Scout Shot oder Scout Video Stream wird von der Exposition Analyse-Einheit (1.2) *lokal im* Intraoral-Röntgensensor (1") analysiert, um Informationen zum Belichtungsgrad zu erfassen. Das Analyseergebnis inklusive der Informationen zum Belichtungsgrad wird mittels der Kommunikationsschnittstelle (1.1) an das Intraoral Röntgengerät (2) bzw. an ein anderes externes Gerät (z.B. ein Rechner) weitergeleitet. Dazu hat das Intraoral-Röntgengerät (2) entsprechende Kommunikationsschnittstellen (2.1.1; 2.5). Das Intraoral-Röntgengerät (2) hat eine Entscheidungs-Einheit (2.2), die das Analyseergebnis inklusive der erfassten Information zum Belichtungsgrad auswertet und über die Abfolge weiterer Belichtungen insbesondere die Anzahl der Schüsse und deren Dauer entscheidet. Im Fall eines Video Streams kann dies auch während der laufenden Belichtung erfolgen. Bei der Verwendung eines Videostreams erweitert sich der erste Shot und zweite Shot auf die Menge von ersten "m" Shots und zweiten "n" Shots. In diesem Fall kann der Prozess auch mehrfach wiederholt werden. Der IO-Röntgensensor (1") bleibt bis zum Ende der Aufnahme-Session in entsprechender Aufnahmebereitschaft. Das Intraoral-Röntgengerät (2) hat ferner einen Röntgenstrahler (2.3), eine Stromversorgung und Hochspannungsquelle (2.6) und eine Steuerung (2.4). Die Entscheidungseinheit (2.2) ist ferner ausgebildet sensorspezifischen Eigenschaften und/oder vom Anwender vorgegebene Bildqualitätsparameter zu berücksichtigen. Die sensorspezifischen Eigenschaften umfassen Angaben u.a. zu Sensor-Dimensionen, zu lokalen Pixelfehlern, zum Dosis/Signal-Verhalten und zum maximalen Sättigungspegel ab dessen Überschreiten Sättigungseffekte eintreten, die keine oder eingeschränkte Digitalisierung des analogen Belichtungssignals erlauben. Die Bildqualitätsparameter können vom Benutzer direkt oder indirekt durch eine Benutzerschnittstelle (2.7) am Röntgengerät (2) eingegeben werden. Eine indirekte Vorgabe kann über die ärztliche Indikation (z.B. Klärung von Verdacht auf Karies, Paradentose, Wurzelverlauf) erfolgen, die vom Intraoral-Röntgensystem (3) mit der Entscheidungseinheit (2.2) in Belichtungsparameter umgesetzt wird, z.B. maximale Belichtung, Dual Energy etc.. Alternativ kann dies auch über eine Benutzerschnittstelle (4.2) eines Rechners (4.1) geschehen, der am Intraoral-Röntgengerät (2) angeschlossen ist. Das Intraoral-Röntgengerät (2) ist mit seiner Kommunikationsschnittstelle (2.5) und vorzugsweise über ein Netzwerk (5) mit dem Rechner (4.1) verbunden. Das Intraoral-Röntgengerät (2) und der Rechner (4.1) können auch eine Verbindung zu einer Cloud (6) haben. Das empfangene Rohbildmaterial wird zu einem ersten Rohbild aufgearbeitet, um sensor- und aufnahmespezifische Unzulänglichkeiten zu kompensieren. Diese sind: Gain, Blemish- und DC-Korrektur (klassisch), Dynamik-Erweiterung (HDR), Bewegungsartefakt-Kompensation (Anti-Shake). Anstelle, dass der IO-Röntgensensor oder das Röntgengerät (2) diese und weitere bildverarbeitende Funktionen unter Verwendung jeweiliger, interner Bildspeicher (2.9;1.6) ausführt, kann dies in der Cloud (6) oder durch den PC (4.1) erfolgen.

Das Intraoral-Röntgengerät (2) verfügt zwar über eine Exposition Analyse-Einheit (2.8), aber diese wird nicht benötigt, weil der IO-Röntgensensor (1") die Auswertung selbst ausführt. Ferner werden die dick andere umrandete Komponenten auch nicht zwingend benötigt. Vorzugsweise werden die Kommunikationsschnittstellen (2.1.1) bis (2.1.3) separat oder alternativ als eine gemeinsame Kommunikationsschnittstelle bereitgestellt.

### AEC Modus

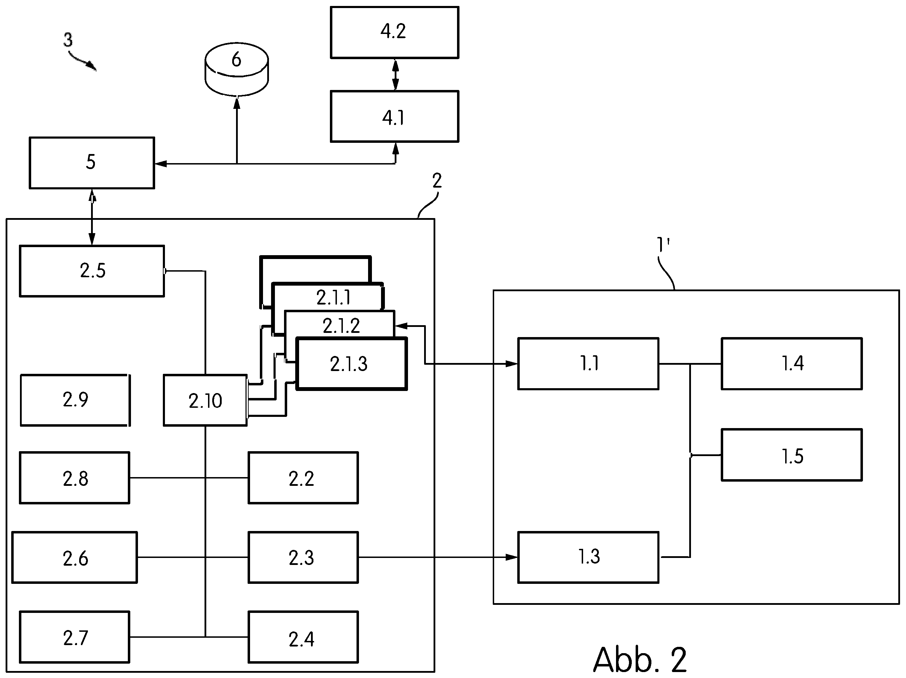

Im Folgenden wird der Modus (M') erklärt, welcher den eingangsseitig erklärten AEC Modus entspricht: Abb.2 zeigt eine schematische Darstellung eines Intraoral-Röntgensystems (3) gemäß der Erfindung. Das Intraoral-Röntgensystem (3) hat ein Intraoral-Röntgengerät (2), das mit einem Intraoral-Röntgensensor (1') über die Kommunikationsstellen (2.1.2;1.1) verbunden ist. Die Verbindung ist vorzugsweise eine Kabel Verbindung. Der IO-Röntgensensor (1') bestehet aus einem Sensorkopf (Gehäuse), Kabel und Stecker. Über diesen Stecker ist der IO-Röntgensensor (1') mit dem Intraoral-Röntgengerät (2) verbunden. Am IO-Röntgengerät (2) können vorzugsweise gleichzeitig weitere Sensoren (1, 1',1") mit oder ohne AEC Funktionalität über deren Stecker angeschlossen sein. Alternativ kann eine drahtlose Verbindung benutzt werden. Der Intraoral-Röntgensensor (1') hat eine Kommunikationsschnittstelle (1.1), *keine* Exposition Analyse-Einheit (1.2), einen bildgebenden Röntgendetektor (1.3), eine Steuerung (1.4), und ein Speicher (1.5), der die Sensorspezifische Eigenschaften beinhaltet. Vorzugsweise kann der Röntgensensor (1') mit einer Batterie und einer drahtlosen Kommunikation ausgestattet sein. Dies erlaubt auf ein Kabel zu verzichten. Eine kabelgebundene Datenkommunikation und/oder Energieversorgung kann auch alternativ berücksichtigt werden. Hier bietet sich als Standard eine USB-Schnittstelle oder Power over Ethernet (PoE) an.

Der Intraoral-Röntgensensor (1') beinhaltet *keine* Komponenten für eine automatische Belichtungskontrolle (AEC) Funktionalität. Der vom bildgebenden Röntgendetektor (1.3) empfangene Scout Shot oder Scout Video Stream wird von der Exposition Analyse-Einheit *(2.8) lokal im* Röntgengerät (2) analysiert, um Informationen zum Belichtungsgrad zu erfassen. Das Intraoral-Röntgengerät (2) hat eine Entscheidungs-Einheit (2.2), die das Analyseergebnis inklusive der erfassten Information zum Belichtungsgrad auswertet und über die Abfolge weiterer Belichtungen insbesondere die Anzahl der Schüsse und deren Dauer entscheidet. Im Fall eines Video Streams kann dies auch während der laufenden Belichtung erfolgen. Bei der Verwendung eines Videostreams erweitert sich der erste Shot und zweite Shot auf die Menge von ersten "m" Shots und zweiten "n" Shots. In diesem Fall kann der Prozess auch mehrfach wiederholt werden. Der IO-Röntgensensor (1') bleibt bis zum Ende der Aufnahme-Session in entsprechender Aufnahmebereitschaft. Das Intraoral-Röntgengerät (2) hat ferner einen Röntgenstrahler (2.3), eine Stromversorgung und Hochspannungsquelle (2.6) und eine Steuerung (2.4). Die Entscheidungseinheit (2.2) ist ferner ausgebildet die sensorspezifischen Eigenschaften und/oder vom Anwender vorgegebene Bildqualitätsparameter zu berücksichtigen. Die Sensorspezifischen Eigenschaften umfassen Angaben u.a. zu Sensor-Dimensionen, zu lokalen Pixelfehlern, zum Dosis/Signal-Verhalten und zum maximalen Sättigungspegel ab dessen Überschreiten Sättigungseffekte eintreten, die keine oder eingeschränkte Digitalisierung des analogen Belichtungssignals erlauben. Die Bildqualitätsparameter können vom Benutzer direkt oder indirekt durch eine Benutzerschnittstelle (2.7) am Röntgengerät (2) eingegeben werden. Eine indirekte Vorgabe kann über die ärztliche Indikation (z.B. Klärung von Verdacht auf Karies, Paradentose, Wurzelverlauf) erfolgen, die vom Intraoral-Röntgensystem (3) mit der Entscheidungseinheit (2.2) in Belichtungsparameter umgesetzt wird, z.B. maximale Belichtung, Dual Energy etc.. Alternativ kann dies auch über eine Benutzerschnittstelle (4.2) eines Rechners (4.1) geschehen, der am Intraoral-Röntgengerät (2) angeschlossen ist. Das Intraoral-Röntgengerät (2) ist mit seiner Kommunikationsschnittstelle (2.5) und vorzugsweise über ein Netzwerk (5) mit einem Rechner (4.1) verbunden. Das Intraoral-Röntgengerät (2) und der Rechner (4.1) können auch eine Verbindung zu einer Cloud (6) haben. Das empfangene Rohbildmaterial wird zu einem ersten Rohbild aufgearbeitet, um sensor- und aufnahmespezifische Unzulänglichkeiten zu kompensieren. Diese sind: Gain, Blemish- und DC-Korrektur (klassisch), Dynamik-Erweiterung (HDR), Bewegungsartefakt-Kompensation (Anti-Shake). Anstelle, dass der Röntgensensor (1') oder das Röntgengerät (2) bildverarbeitende Funktionen ausführt, kann dies in der Cloud (6) oder durch den PC (4.1) erfolgen. Das Röntgengerät (2) hat auch einen Bildspeicher (2.9).

Die Kommunikationsschnittstellen (2.1.1) bis (2.1.3) werden vorzugsweise separat oder alternativ als eine gemeinsame Kommunikationsschnittstellen bereitgestellt.

### Standard Modus

Im Folgenden wird der Modus (M) erklärt, welcher den eingangsseitig erklärten Standard Modus entspricht: Abb.1 zeigt eine schematische Darstellung eines Intraoral-Röntgensystems (3) gemäß eines Ausführungsbeispiels der Erfindung. Das Intraoral-Röntgensystem (3) hat ein Intraoral-Röntgengerät (2), das mit einem Intraoral-Röntgensensor (1) über die Kommunikationsstellen (2.1.3; 1.1) verbunden ist. Die Verbindung ist vorzugsweise eine Kabel Verbindung. Der IO-Röntgensensor (1) bestehet aus einem Sensorkopf (Gehäuse), Kabel und Stecker. Über diesen Stecker ist der IO-Röntgensensor (1) mit dem Intraoral-Röntgengerät (2) verbunden. Am IO-Röntgengerät (2) können vorzugsweise gleichzeitig weitere IO Röntgensensoren (1, 1',1") mit oder ohne AEC Funktionalität über deren Stecker angeschlossen sein. Alternativ kann eine drahtlose Verbindung benutzt werden. Der Intraoral-Röntgensensor (1) hat eine Kommunikationsschnittstelle (1.1), *keine* Exposition Analyse-Einheit (1.2), einen bildgebenden Röntgendetektor (1.3), eine Steuerung (1.4), ein Speicher (1.5), der die sensorspezifische Eigenschaften beinhaltet. Vorzugsweise kann der IO-Röntgensensor (1) mit einer Batterie und einer drahtlosen Kommunikation ausgestattet sein. Dies erlaubt auf ein Kabel zu verzichten. Eine kabelgebundene Datenkommunikation und/oder Energieversorgung kann auch alternativ berücksichtigt werden. Hier bietet sich als Standard eine USB-Schnittstelle oder Power over Ethernet (PoE) an.

Der Intraoral-Röntgensensor (1) beinhaltet *keine* Komponenten für eine automatische Belichtungskontrolle (AEC) Funktionalität. Der IO-Röntgensensor (1) bleibt bis zum Ende der Aufnahme-Session in entsprechender Aufnahmebereitschaft. Das Intraoral-Röntgengerät (2) hat ferner einen Röntgenstrahler (2.3), eine Stromversorgung und Hochspannungsquelle (2.6) und eine Steuerung (2.4).

Das Intraoral-Röntgengerät (2) ist mit seiner Kommunikationsschnittstelle (2.5) und vorzugsweise über ein Netzwerk (5) mit einem Rechner (4.1) verbunden. Das Intraoral-Röntgengerät (2) und der Rechner (4.1) können auch eine Verbindung zu einer Cloud (6) haben. Anstelle, dass der Röntgensensor (1) oder das Röntgengerät (2) bildverarbeitende Funktionen ausführt, kann dies in dem PC (4.1) erfolgen. Das Röntgengerät (2) kann auch einen Bildspeicher (2.9) haben. Das empfangene Rohbildmaterial wird zu einem ersten Rohbild aufgearbeitet, um sensor- und aufnahmespezifische Unzulänglichkeiten zu kompensieren. Diese sind: Gain, Blemish- und DC-Korrektur (klassisch), Dynamik-Erweiterung (HDR), Bewegungsartefakt-Kompensation (Anti-Shake).

Das Intraoral-Röntgengerät (2) verfügt zwar über eine Exposition Analyse-Einheit (2.8), aber diese wird in dieser Konstellation nicht benötigt. Ferner werden die andere dick umrandete Komponenten auch nicht zwingend benötigt.

Die Kommunikationsschnittstellen (2.1.1) bis (2.1.3) können vorzugsweise separat oder alternativ als eine gemeinsame Kommunikationsschnittstelle bereitgestellt werden.

In der vorliegenden Erfindung wurden drei Modi vorgestellt. Vorstellbar sind auch weitere Modi mit zugehörigen Sensortypen. Weitere Kommunikationsschnittstellen können separat hinzugefügt werden. Je nach deren Funktionalität werden mehr oder weniger Funktionskomponenten des Röntgengerätes (2) oder Röntgensystems (3) ansprechbar. Die Funktionsweiche (2.10) identifiziert den jeweiligen Sensortyp, aktiviert den entsprechenden Modus Modi (M; M'; M"), um die erweiterten Funktionskomponenten auch einzubinden.

## Patentansprüche

1. Intraoral-Röntgensystem (3) umfassend:
ein Intraoral-Röntgengerät (2), das mit einer automatischen Belichtungskontrolle (AEC) Funktionalität ausgestattet ist, und mit einer Cloud (6) und einem Rechner (4.1) verbindbar ist,
wobei an dem Intraoral-Röntgengerät (2) mindestens ein Intraoral-Röntgensensor (1;1';1") über Kommunikationsstellen (2.1.2; 1.1) anschließbar ist, wobei das Intraoral-Röntgengerät (2) eine Exposition Analyse-Einheit (2.8) aufweist, welche geeignet ist, Information über den Belichtungsgrad eines vom Intraoral-Röntgensensor über die Kommunikationsstellen weitergeleiteten Scout Bildes oder Video Streams zu erfassen und diese an eine Entscheidungs-Einheit (2.2) des Röntgengerätes (2), Rechners (4.1) und/oder der Cloud (4) zur Auswertung und Entscheidung weiterer Belichtungen weiterzuleiten, und
wobei das Intraoral-Röntgengerät (2) eine Funktionsweiche (2.10) aufweist, welche in einem initialen Identifikationsprozess den Sensortyp des mindestens einem angeschlossenen intraoralen Röntgensensors (1;1';1") erkennt und einen dazugehörigen Modus (M; M'; M") aktiviert, wobei das Intraoral-Röntgensystem (3) die folgenden Modi (M; M'; M") umfasst: ein Modus (M") zur Intraoral-Röntgensensorseitigen Erfassung eines Belichtungsgrades, wobei der Sensortyp für den Modus (M") zur Intraoral-Röntgensensorseitigen Erfassung eines Belichtungsgrades ein Intraoral-Röntgensensor (1") mit Exposition Analyse-Einheit (1.2) ist, und ein Modus (M') zur Intraoral-Röntgengerät-, Rechner- oder Cloudseitigen Erfassung eines Belichtungsgrades, wobei der Sensortyp für den Modus (M') zur Intraoral-Röntgengerät-, Rechner- oder Cloudseitigen Erfassung eines Belichtungsgrades ein Intraoral-Röntgensensor (1') ohne Exposition Analyse-Einheit ist.

2. Intraoral-Röntgensystem (3) nach Anspruch 1, wobei das System außerdem einen, dem Modus (M") zur Intraoral-Röntgensensorseitigen Erfassung eines Belichtungsgrades zugehörigen, Intraoral-Röntgensensor (1") aufweist, der eine Exposition Analyse-Einheit (1.2), einen bildgebenden Röntgendetektor (1.3), und eine Kommunikationsschnittstelle (1.1) beinhaltet, wobei der vom bildgebenden Röntgendetektor (1.3) empfangene Scout Shot oder Scout Video Stream von der Exposition Analyse-Einheit (1.2) im Intraoral-Röntgensensor (1") analysiert wird, um Informationen über den Belichtungsgrad des Scout Bildes oder Video Streams zu erfassen und um diese mittels der Kommunikationsschnittstelle (1.1) an eine vom Intraoral-Röntgensensor (1) extern angeordnete Entscheidungseinheit (2.2) des Intraoral-Röntgengeräts (2) oder eines anderen Gerätes zur Auswertung und Entscheidung weiterer Belichtungen weiterzuleiten.

3. Intraoral-Röntgensystem (3) nach einem der Ansprüche 1 bis 2, wobei das System außerdem einen, dem Modus (M') zur Intraoral-Röntgengerät-, Rechner-, oder Cloudseitigen Erfassung eines Belichtungsgrades zugehörigen, Intraoral-Röntgensensor (1') aufweist, der keine Informationen zum Belichtungsgrad erfasst, und der einen bildgebenden Röntgendetektor (1.3) und eine Kommunikationsschnittstelle (1.1) beinhaltet, wobei der vom bildgebenden Röntgendetektor (1.3) empfangene Scout Shot oder Video Stream ans Röntgengerät (2) mittels der Kommunikationsschnittstelle (1.1) weitergeleitet und von der Exposition Analyse-Einheit (2.8) im Röntgengerät (2) lokal analysiert wird, um Information über den Belichtungsgrad des Scout Bildes oder Video Streams zu erfassen und diese an eine Entscheidungs-Einheit (2.2) des Röntgengerätes (2), Rechners (4.1) und/oder der Cloud (4) zur Auswertung und Entscheidung weiterer Belichtungen weiterleitet wird.

4. Intraoral-Röntgensystem (3) nach einem der Ansprüche 1 bis 3, wobei es ferner einen Modus (M) ohne Erfassung eines Belichtungsgrades umfasst.

5. Intraoral-Röntgensystem (3) nach Anspruch 4, wobei das System außerdem einen, dem Modus (M) ohne Erfassung eines Belichtungsgrades zugehörigen, Intraoral-Röntgensensor (1) aufweist, der keine Informationen zum Belichtungsgrad erfasst, und wobei für den Modus (M) das Röntgengerät (2) auch keine Informationen zum Belichtungsgrad erfasst.

## Claims

1. Intraoral X-ray system (3) comprising:
an intraoral X-ray device (2), which is equipped with an automatic exposure control (AEC) functionality and is connectable to a cloud (6) and a computer (4.1),
wherein at least one intraoral X-ray sensor (1; 1'; 1") can be connected to the intraoral X-ray device (2) via communication points (2.1.2; 1.1), wherein the intraoral X-ray device (2) has an exposure analysis unit (2.8) which is suitable for recording information about the exposure level of a scout image or video stream forwarded by the intraoral X-ray sensor via the communication points and for forwarding this information to a decision-making unit (2.2) of the X-ray device (2), computer (4.1) and/or cloud (4) for evaluation and decision-making on further exposures, and
wherein the intraoral X-ray device (2) has a function switch (2.10) which, in an initial identification process, recognizes the sensor type of the at least one connected intraoral X-ray sensor (1; 1'; 1") and activates an associated mode (M; M'; M"), wherein the intraoral X-ray system (3) comprises the following modes (M; M'; M"): a mode (M") for the intraoral X-ray sensor-side detection of an exposure level, wherein the sensor type for mode (M") for the intraoral X-ray sensor-side detection of an exposure level is an intraoral X-ray sensor (1") with an exposure analysis unit (1.2), and a mode (M') for the intraoral X-ray device-side, computer-side or cloud-side detection of an exposure level, wherein the sensor type for mode (M') for the intraoral X-ray device-side, computer-side or cloud-side detection of an exposure level is an intraoral X-ray sensor (1') without an exposure analysis unit.

2. Intraoral X-ray system (3) according to claim 1, wherein the system further has an intraoral X-ray sensor (1") associated with mode (M") for the intraoral X-ray sensor-side detection of an exposure level which includes an exposure analysis unit (1.2), an imaging X-ray detector (1.3), and a communication interface (1.1), wherein the scout shot or scout video stream received from the imaging X-ray detector (1.3) is analyzed by the exposure analysis unit (1.2) in the intraoral X-ray sensor (1") in order to record information about the exposure level of the scout image or video stream and to forward this information by means of the communication interface (1.1) to a decision-making unit (2.2) of the intraoral X-ray device (2) or another device, which decision-making unit is arranged externally of the intraoral X-ray sensor (1), for evaluation and decision-making on further exposures.

3. Intraoral X-ray system (3) according to one of claims 1 to 2, wherein the system further has an intraoral X-ray sensor (1') associated with mode (M') for the intraoral X-ray device-side, computer-side or cloud-side detection of an exposure level which does not record any information on the exposure level and which includes an imaging X-ray detector (1.3) and a communication interface (1.1), wherein the scout shot or video stream received from the imaging X-ray detector (1.3) is forwarded to the X-ray device (2) by means of the communication interface (1.1) and analyzed locally by the exposure analysis unit (2.8) in the X-ray device (2), to record information about the exposure level of the scout image or video stream and forward this to a decision-making unit (2.2) of the X-ray device (2), computer (4.1) and/or the cloud (4) for evaluation and decision-making on further exposures.

4. Intraoral X-ray system (3) according to one of claims 1 to 3, wherein it further comprises a mode (M) without detection of an exposure level.

5. Intraoral X-ray system (3) according to claim 4, wherein the system further has an intraoral X-ray sensor (1) associated with mode (M) without detection of an exposure level which does not record any information on the exposure level, and wherein for mode (M) the X-ray device (2) also does not record any information on the exposure level.

## Revendications

1. Système de radiographie intra-orale (3) comprenant :
un appareil de radiographie intra-orale (2), qui est doté d'une fonctionnalité de contrôle automatique de l'exposition (AEC) et qui peut être connecté à un nuage (6) et à un ordinateur (4.1),
au moins un capteur pour radiographie intra-orale (1 ; 1' ; 1") étant raccordable à l'appareil de radiographie intra-orale (2) par l'intermédiaire de points de communication (2.1.2 ; 1.1), l'appareil de radiographie intra-orale (2) comprenant une unité d'analyse d'exposition (2.8) qui est apte à saisir des informations sur le degré d'exposition d'une image Scout ou d'un flux vidéotransmis par le capteur pour radiographie intra-orale par l'intermédiaire des points de communication et à les transmettre à une unité de décision (2.2) de l'appareil de radiographie (2), de l'ordinateur (4.1) et/ou du nuage (4) pour l'évaluation et la décision d'autres expositions, et
l'appareil de radiographie intra-orale (2) comprenant un aiguillage fonctionnel (2.10) qui, dans un processus d'identification initial, reconnaît le type de capteur d'au moins un capteur pour radiographie intra-orale raccordé (1 ; 1' ; 1") et active un mode correspondant (M ; M' ; M"), le système de radiographie intra-orale (3) comprenant les modes suivants (M ; M' ; M") : un mode (M") pour la détection côté capteur pour radiographie intra-orale d'un degré d'exposition,le type de capteur pour le mode (M") pour la détection côté capteur pour radiographie intra-orale d'un degré d'exposition étant un capteur pour radiographie intra-orale (1") avec unité d'analyse d'exposition (1.2), et un mode (M') pour la détection côté appareil de radiographie intra-orale, ordinateur ou nuage d'un degré d'exposition, le type de capteur pour le mode (M') pour la détection côté appareil de radiographie intra-orale, ordinateur ou nuage d'un degré d'exposition étant un capteur pour radiographie intra-orale (1') sans unité d'analyse d'exposition.

2. Système de radiographie intra-orale (3) selon la revendication 1, le système comprenant en outre un capteur pour radiographie intra-orale (1") associé au mode (M") pour la détection côté capteur pour radiographie intra-orale d'un degré d'exposition, qui comprend une unité d'analyse d'exposition (1.2), un détecteur de rayons X d'imagerie (1.3) et une interface de communication (1.1), le scout shot ou le flux vidéo de balayage reçu par le détecteur de rayons X d'imagerie (1.3) étant analysé par l'unité d'analyse d'exposition (1.2) dans le capteur pour radiographie intra-orale (1") afin de saisir des informations sur le degré d'exposition de l'image Scout ou du flux vidéo et de les transmettre au moyen de l'interface de communication (1.1) à une unité de décision (2.2) de l'appareil de radiographie intra-orale (2) ou d'un autre appareil, disposée à l'extérieur du capteur pour radiographie intra-orale (1), pour évaluer et décider d'autres expositions.

3. Système de radiographie intra-orale (3) selon l'une des revendications 1 à 2, le système comprenant en outre un capteur pour radiographie intra-orale (1') associé au mode (M') pour la détection côté appareil de radiographie intra-orale, ordinateur ou nuage d'un degré d'exposition, qui ne saisit pas d'informations sur le degré d'exposition et qui comprend un détecteur de rayons X d'imagerie (1.3) et une interface de communication (1.1), le scout shot ou le flux vidéo reçu par le détecteur de rayons X d'imagerie (1.3) étant transmis à l'appareil de radiographie (2) au moyen de l'interface de communication (1.1) et analysé localement par l'unité d'analyse d'exposition (2.8) dans l'appareil de radiographie (2), afin de saisir des informations sur le degré d'exposition de l'image Scout ou du flux vidéo et de les transmettre à une unité de décision (2.2) de l'appareil de radiographie (2), de l'ordinateur (4.1) et/ou du nuage (4) pour évaluer et décider d'autres expositions.

4. Système de radiographie intra-orale (3) selon l'une des revendications 1 à 3, celui-ci comprenant en outre un mode (M) sans détection d'un degré d'exposition.

5. Système de radiographie intra-orale (3) selon la revendication 4, le système comprenant en outre un capteur pour radiographie intra-orale (1) associé au mode (M) sans détection d'un degré d'exposition, qui ne détecte pas d'informations sur le degré d'exposition, et dans lequel, pour le mode (M), l'appareil de radiographie (2) ne détecte pas non plus d'informations sur le degré d'exposition.
